# EUROPÄISCHE PATENTSCHRIFT

(11) **EP 0 827 737 B1**
(45) Veröffentlichungstag und Bekanntmachung des Hinweises auf die Patenterteilung: **06.12.2000**
(21) Anmeldenummer: 97115057.8
(22) Anmeldetag: 30.08.1997
(51) Int. Cl.: A61K 7/06, A61K 7/135, A61K 7/09, A61K 7/13

(54) **Verfahren zur Aufhellung, Glanzverbesserung und Färbung von menschlichen Haaren**
Method for lightening, improving the gloss of and colouring human hair
Procédé pour blanchir, pour améliorer la brillance et pour colorer les cheveux humains

(30) Priorität: 04.09.1996 DE 19635877
(43) Veröffentlichungstag der Anmeldung: 11.03.1998
(73) Patentinhaber: GOLDWELL GmbH, D-64297 Darmstadt (DE)
(72) Erfinder: Golinski, Frank, Dr., 64297 Darmstadt (DE); Lorenz, Heribert, 64401 Gross-Bieberau (DE); Kufner, Frank, 64297 Darmstadt (DE)

(56) Entgegenhaltungen:
- EP-A- 0 133 905
- EP-A- 0 260 716
- EP-A- 0 308 825
- DE-A- 4 017 718
- US-A- 4 130 501
- US-A- 5 340 367
- PATENT ABSTRACTS OF JAPAN, Band 199, Nr. 610, 31. Oktober 1996 & JP 08 157345 A

## Beschreibung

Die vorliegende Erfindung betrifft die Verwendung eines Haarbehandlungsmittels, insbesondere bei der abschließenden Behandlung dauergewellten Haares, der sogenannten Schlußfixierung, wobei eine aufhellende, glanzverbessernde Wirkung auf das Haar ausgeübt wird.

Gemäß einer bevorzugten Ausführungsform kann durch die Anwendung eines erfindungsgemäß verwendeten Mittels durch Zusatz eines direktziehenden Haarfarbstoffs auch eine besonders brillante Haarfärbung erzielt werden.

Das erfindungsgemäß verwendete Mittel enthält 0,5 bis 12 Gew.-%, berechnet auf die Gesamtzusammensetzung, Wasserstoffperoxid, in einer wäßrigen Grundlage und weist eine Viskosität von 1000 bis 10 000 mPa.s bei 25 °C, gemessen im Brookfield-Viskosimeter RVT mit Spindel Nr. 4 bei 20 rpm, und einen pH-Wert von 3 bis 7 auf.

Der bevorzugte Viskositätsbereich liegt dabei bei etwa 1500 bis 5000 mPa.s bei 25 °C, insbesondere 2000 bis 4000.

Gemäß einer weiteren bevorzugten Ausführungsform der Erfindung enthält das erfindungsgemäß verwendete Mittel 0,001 bis 2,5, insbesondere 0,01 bis 1,5, besonders bevorzugt 0,1 bis 1 Gew.-%, berechnet auf die Gesamtzusammensetzung, mindestens eines direktziehenden Haarfarbstoffs. Die bevorzugte Menge an Wasserstoffperoxid liegt bei 2 bis 8, insbesondere 2,5 bis 6 Gew.-%, berechnet auf die Gesamtzusammensetzung des Mittels.

Die Erfindung betrifft ferner ein Verfahren zum Aufhellen, Glanzgeben und gegebenenfalls Färben von dauergewelltem Haar, wobei auf das mit einer Reduktionsmittel-Zusammensetzung verformte Haar nach dem Spülen mit Wasser, gegebenenfalls nach Durchführung einer Zwischenbehandlung, in an sich üblicher Weise das Haar durch Aufbringung einer Oxidationsmittel-Zusammensetzung fixiert, anschließend eine erfindungsgemäß zu verwendende Zusammensetzung aufgebracht und nach etwa fünf- bis etwa dreißigminütiger Einwirkung ausgespült wird.

Durch diese Behandlung wird eine glänzende Aufhellung des Haares und, bei Anwesenheit eines direktziehenden Haarfarbstoffs, auch eine brillante Haarfärbung erreicht.

Alternativ dazu kann die erfindungsgemäß verwendete Zusammensetzung auch zur direkten Fixierung des dauergewellten Haares benutzt oder auf das gewaschene bzw. gespülte Haar im Rahmen einer Haarbehandlung ohne vorhergehende Dauerwelle aufgebracht werden.

Die üblicherweise für die Fixierung dauergewellten Haares eingesetzten Oxidationsmittel-Zusammensetzungen auf Basis von Peroxiden oder Alkalibromaten weisen Viskositäten von weniger als 1000 mPa.s, zumeist weniger als 100 mPa.s, z. B. zwischen 2,5 und 50 mPa.s, auf, vgl. z. B. die DE-A 41 31 992; mit ihnen läßt sich die erfindungsgemäße Wirkung nicht erzielen.

Ohne sich auf einen speziellen Wirkungsmechanismus festlegen zu wollen, erscheint es möglich, daß durch die Aufbringung der höherviskosen Zusammensetzung ein intensiverer Haarkontakt ermöglicht wird, der zu einer verbesserten Einwirkung führt. Auch ist dadurch die Mitverwendung zusätzlicher Haarpflege- und Konditioniersubstanzen möglich.

Geeignete Verdickungsmittel in den erfindungsgemäß verwendeten Mitteln sind beispielsweise natürliche und synthetische Polymere wie beispielsweise Cellulosederivate wie Hydroxyalkylcellulosen, Galactomannan-Polysaccharide wie Guar Gum und dessen Derivate, Xanthan Gum, Maltodextrine, Acrylsäurehomo- und -copolymerisate, insbesondere des Typs "Carbomer" etc., oder auch die bekannten anorganischen Verdickungsmittel.

Diese Verdickungsmittel sind vorzugsweise in einer Menge von etwa 0,1 bis etwa 5 Gew.-%, insbesondere etwa 0,25 bis etwa 2,5 Gew.-%, berechnet auf die Gesamtzusammensetzung des Mittels, enthalten; ihre Menge ist natürlich abhängig von der gewünschten Viskosität und der sonstigen Zusammensetzung des Mittels.

Bevorzugte Cellulosederivate sind insbesondere Hydroxyethyl-, Hydroxypropyl- und Hydroxymethylpropylcellulose; bevorzugte Guar-Derivate sind Hydroxypropyl-Guar, d.h., der Propylenglykolether des Guar Gums, sowie Quaternisierungsprodukte desselben, insbesondere das Hydroxypropyl-Guar-hydroxypropyltrimoniumchlorid. Weitere geeignete Hydroxyalkyl-Guar-Derivate sind beispielsweise Hydroxyethyl-Guar, Hydroxybutyl-Guar und deren Quaternisierungsprodukte.

Geeignete Handelsprodukte sind unter den Markennamen "Jaguar HP™", "Jaguar C-17™" und "Jaguar C-162™" sowie "Galactosol™" auf dem Markt.

Liegt die erfindungsgemäß verwendete Zusammensetzung nicht als wäßriges Gel, sondern als Emulsion bzw. Dispersion vor, kann die Viskosität durch das Verhältnis Emulgatoren/Co-Emulgatoren, beispielsweise nichtionische Polyoxyethylenderivate und Fettalkohole, in an sich bekannter Weise eingestellt werden.

Auch Fettsäurealkanolamide sind, wie ebenfalls bekannt, als viskositätssteigernde Verbindungen für diesen Zweck einsetzbar.

Als direktziehende Haarfarbstoffe können im Prinzip alle für diesen Zweck zugelassenen Farbstoffe verwendet werden; es wird hierzu insbesondere auf die deutsche "Verordnung über kosmetische Mittel (Kosmetik-Verordnung)" in der jeweils geltenden Fassung, Anlage 3, verwiesen.

Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Basic Green 1, | C.I.-No. 42,040; |
| Basic Red 2, | C.I.-No. 50,240; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, und | C.I.-No. 42,510; |
| Basic Yellow 57, | C.I.-No. 12,719. |

Als mögliche saure (anionische) Farbstoffe können Verwendung finden:

| | |
|---|---|
| Acid Black 1, | C.I.-No. 20,470; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.I.-No. 45,380; |
| Acid Red 92, | C.I.-No. 45,410; |
| Acid Violet 43, | C.I.-No. 60,730; |
| Acid Yellow 1, | C.I.-No. 10,316; |
| Acid Yellow 23, | C.I.-No. 19,140; |
| Acid Yellow 3, | C.I.-No. 47,005; |
| D&C Brown No.1, | C.I.-No. 20,170; |
| D&C Green No.5, | C.I.-No. 61,570; |
| D&C Orange No.4, | C.I.-No. 15,510; |
| D&C Orange No.10, | C.I.-No. 45,425:1; |
| D&C Orange No.11, | C.I.-No. 45,425; |
| D&C Red No.21, | C.I.-No. 45,380:2; |
| D&C Red No.27, | C.I.-No. 45,410:1; |
| D&C Red No.33, | C.I.-No. 17,200; |
| D&C Yellow No.7, | C.I.-No. 45,350:1; |
| D&C Yellow No.8, | C.I.-No. 45,350; |
| FD&C Red No.4, | C.I.-No. 14,700; |
| FD&C Yellow No.6, | C.I.-No. 15,985. |

Auch pflanzliche Farbstoffe können allein oder mit synthetischen Direktziehern Verwendung finden, beispielsweise Henna (rot oder schwarz), Alkannawurzel, Laccainsäure (Stocklack), Blauholzpulver, Krappwurzel- und Rhabarberwurzelpulver.

Die nach der Erfindung verwendeten Mittel enthalten vorzugsweise noch mindestens ein synthetisches oder natürliches haarkonditionierendes Polymeres, insbesondere in einer Menge von 0,1 bis 2,5, insbesondere 0,25 bis 1,5 Gew.-% der Gesamtzusammensetzung, Grundsätzlich können alle Arten von Polymeren verwendet werden, also nichtionische, anionische, amphotere und kationische Polymere, natürlich in Abhängigkeit von deren Verträglichkeit mit den weiteren Ingredienzien der Zusammensetzung wie Farbstoffen und Tensiden.

Geeignete kationische Polymere sind neben den altbekannten quaternären Cellulosederivaten des Typs "Polymer JR" insbesondere quaternisierte Homo- und Copolymere des Di-methyldiallylammoniumchlorids, wie sie unter dem Handelsnamen "Merquat" im Handel sind, quaternäre Vinylpyrrolidon-Copolymere, insbesondere mit Dialkylaminoalkyl(meth)acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind, geeignet.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Anstelle der kationischen Polymeren oder in Kombination mit denselben können auch nichtionische Polymere eingesetzt werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat. wie sie beispielsweise von der Firma BASF unter dem Handelsnamen "Luviskol" vertrieben werden, eingesetzt.

Es können jedoch auch (Co-)Polymerisate aus den verschiedenen Acryl- und Methacrylestern, Acrylamid und Methacrylamid, beispielsweise Polyacrylamid mit Molgewichten von über 100.000, Dimethylhydantoin-Formaldehyd-Harze, etc., eingesetzt werden. Selbstverständlich sind auch Mischungen aus verschiedenen nichtionischen Polymeren verwendbar.

Geeignete anionische Polymere im Rahmen der Erfindung sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez AN oder ES" vertrieben werden. Diese Polymeren können auch teilverestert sein, beispielsweise "Gantrez ES 225", der Ethylester eines Ethylvinylether/Maleinsäure-Copolymers, oder der Butyl- oder Isobutylester desselben.

Weitere geeignete anionische Polymere sind insbesondere Vinylacetat/Crotonsäure- oder Vinylacetat/Vinylneodecanoat/Crotonsäure-Copolymere des Typs "Resyn"; Natriumacrylat/Vinylalkohol-Copolymere des Typs "Hydagen F", Natriumpolystyrolsulfonat, z.B. "Flexan 130"; Ethylacrylat/Acrylsäure/N-tert.-Butylacrylamid-Copolymere des Typs "Ultrahold"; Vinylpyrrolidon/Vinylacetat/Itaconsäure-Copolymere, Acrylsäure/Acrylamid-Copolymere bzw. Natriumsalze derselben vom Typ "Reten"; etc.

Grundsätzlich können alle für den Einsatz in Haarpflegemitteln vorgeschlagenen anionischen Polymeren verwendet werden, vorbehaltlich ihrer Verträglichkeit mit den anderen Bestandteilen des erfindungsgemäß verwendeten Mittels.
Als amphotere Polymere, die entweder alternativ zu oder im Gemisch mit den anderen, insbesondere kationischen Polymeren zum Einsatz gelangen, seien Copolymerisate aus N-Octylacrylamid, (Meth)Acrylsäure und tert.-Butylaminoethylmethacrylat vom Typ "Amphomer"; Copolymerisate aus Methacryloylethylbetain und Alkylmethacrylaten vom Typ "Yukaformer", z.B. das Butylmethacrylat-Copolymere "Yukaformer AM75"; Copolymerisate aus Carboxylgruppen oder Sulfogruppen enthaltenden Monomeren, z.B. (Meth)Acrylsäure und Itaconsäure, mit basische, insbesondere Aminogruppen enthaltenden Monomeren wie Mono- und Dialkylaminoalkyl(meth)acrylaten bzw. Mono- und Dialkylaminoalkyl(meth)acrylamiden; Copolymere aus N-Octylamid, Methylmethacrylat. Hydroxypropylmethacrylat, N-tert.-Butylaminoethylmethacrylat und Acrylsäure sowie die aus der US-A 3,927,199 bekannten Copolymeren genannt.

Die erfindungsgemäß verwendeten Haarglanzmittel können die in solchen Zusammensetzungen üblichen Zusätze enthalten, deren Art und Charakter von der Applikationsform des Mittels abhängig ist. Es sind dies Tenside, insbesondere anionische Tenside wie Alkyl(ether)sulfate, Polyethercarbonsäuren, Alkylamidoethercarbonsäuren, langkettige N-Acylaminocarbonsäuren und deren Salze wie N-Lauroylsarkosinat und -glutamat, amphotere Tenside wie Betaine, z. B. Cocoamidopropylbetain, sowie nichtionische und kationische Tenside, Fette, Fettalkohole, Emulgatoren, pH-Regulatoren, Lösungs- und Verbindungsmittel, Lösungsvermittler, Konservierungsmittel, und Parfums.

Der pH-Wert der erfindungsgemäß verwendeten Zusammensetzungen liegt im Bereich von 3 bis 7, vorzugsweise 4 bis 6.

Aus dem Referat der JP 08 157 345 A in den Patent Abstracts of Japan ist ein Verfahren zum Entfärben und Färben von Haar bekannt, wobei eine aus zwei Komponenten hergestellte Zusammensetzung auf das Haar aufgebracht wird, die einen sauren Farbstoff und Wasserstoffperoxid enthält sowie einen pH-Wert über 9,0 aufweist.

Die US 4 130 501 A beschreibt die Verwendung von viskosen Wasserstoffperoxid-Bleichmitteln zum Entfernen von hartnäckigen Flecken.

Die EP 0 308 825 A betrifft eine Öl-in-Wasser-Emulsion, die Wasserstoffperoxid enthält und vor ihrer Anwendung auf dem Haar mit einer alkalisch eingestellten Oxidationshaarfärbe- oder Blondiercreme vermischt und die erhaltene Mischung mit einem pH-Wert von 8 bis 11 auf das Haar aufgebracht wird.

In der EP 0 133 905 A wird eine kationische Zusammensetzung zum Konditionieren von Haaren beschrieben, die als wesentlichen Bestandteil ein Aminoxid enthält.

Aus der DE 40 17 718 A sind schließlich Oxidationshaarfärbemittel bekannt, wobei die das Oxidationsfarbstoffvorprodukt enthaltende Zusammensetzung unmittelbar vor der Anwendung mit einer viskosen, verdickten Wasserstoffperoxid-Zusammensetzung vermischt wird. Die gebrauchsfertige Farbmischung weist einen pH-Bereich von 7,5 bis 12,0, vorzugsweise 9,5 und 10,2 auf.

Die folgenden Beispiele erläutern die Erfindung und deren Effekt im Detail.

Es wurde eine Grundrezeptur der folgenden Zusammensetzung I hergestellt:

| | |
|---|---|
| Cetylstearylalkohol | 3,0 (Gew.-%) |
| Natriumcetylstearylsulfat | 0,3 |
| Stearinsäuremonoethanolamid | 0,5 |
| Kokosfettsäuremonoethanolamid | 0,6 |
| Kokosfettsäuremonoethanolamid, ethoxyliert mit 4 Mol EO | 0,2 |
| Hydroxyethylcellulose | 0,2 |
| Natriumlaurylsulfat | 0,2 |
| Komplexbildner | 0,1 |
| Ammoniumchlorid | 0,1 |
| Ascorbinsäure | 0,1 |
| Roßkastanienextrakt | 0,2 |
| Weizenproteinhydrolysat | 0,2 |
| Parfumöl | 0,2 |
| Wasserstoffperoxid | 4,0 |
| Wasser | @ 100,0 |
| pH-Wert: 4,5 Viskosität bei 25 °C: 2000 mPa.s (gemessen im Brookfield-Viskosimeter RVT mit Spindel Nr. 4 bei 20 rpm). | |

### Beispiel 1

Die Zusammensetzung 1 wurde auf eine angefeuchtete dunkelbraune Strähne aus Menschenhaar aufgetragen und nach dreißigminütiger Einwirkung ausgespült und getrocknet.

Die Strähne wies einen mittelbraunen Glanz auf.

In einem Vergleichsversuch wurde eine gleiche Haarsträhne mit einer analogen Zusammensetzung behandelt, die keine Hydroxyethylcellulose enthielt und durch Herabsetzung des Fettsäuremonoethanolamid-Anteils lediglich eine Viskosität von 250 mPa.s bei 25 °C aufwies.

Das Aussehen der Strähne war nach der Behandlung unverändert.

### Beispiel 2

Die Zusammensetzung I, die zusätzlich 0,1 Gew.-% des direktziehenden Haarfarbstoffs Disperse Violet 4 (unter Abzug von 0,1 Gew.-% Wasser) enthielt, wurde im Halbseitenversuch auf eine Hälfte dauergewellten und mit einer üblichen 6%igen wasserstoffperoxid-Lösung vorfixierten Haares einer Probandin, das eine graumelierte Farbe aufwies, aufgetragen.

Die andere Haarhälfte wurde mit einer analogen Zusammensetzung, deren Viskosität jedoch nur etwa 100 mPa.s bei 25 °C betrug, behandelt.

Nach 15minütiger Einwirkung wurde gespült, getrocknet und das Ergebnis begutachtet.

Die mit der erfindungsgemäßen Zusammensetzung behandelte Haarhälfte zeigte eine glänzende Silberfärbung, während die Vergleichszusammensetzung zu einer stumpfen Graufärbung führte.

### Beispiel 3

Der Zusammensetzung I wurde, unter entsprechender Verringerung des Wasseranteils, ein Gemisch aus direktziehenden Haarfarbstoffen zugesetzt:

| | |
|---|---|
| Basic Bluee 99 | 0,01 (Gew.-%) |
| Basic Brown 16 | 0,02 |
| Basic Brown 17 | 0,03 |
| Basic Yellow 57 | 0,04 |

Diese erfindungsgemäße Zusammensetzung wurde im Halbseitenversuch auf feuchtes, mittelblondes Haar aufgetragen; auf die andere Haarhälfte wurde eine ansonsten identische Zusammensetzung mit einer auf < 100 mPa.s bei 25 °C eingestellten Viskosität aufgebracht.

Nach 15minütiger Einwirkung wurde gespült, getrocknet und das Aussehen beider Haarhälften verglichen.

Während die mit der erfindungsgemäßen Zusammensetzung I behandelte Haarhälfte eine hell glänzende, goldgelbe Färbung aufwies, zeigte die mit der Vergleichszusammensetzung einen matten Blondton.

### Beispiel 4

In einem weiteren Halbseitenversuch wurde die eine Haarhälfte einer Probandin mit mittelbraunem Haar mit der Zusammensetzung I, die die folgende Kombination aus direktziehenden Haarfarbstoffen enthielt, behandelt:

| | |
|---|---|
| Basic Blue 99 | 0,12 (Gew.-%) |
| Basic Brown 16 | 0,17 |
| Basic Brown 17 | 0,07 |
| HC Yellow 5 | 0,12 |
| HC Red 3 | 0,20 |
| (Gewichtsausgleich durch Reduktion des Wassergehalts). | |

Die andere Haarhälfte wurde mit einer analogen Zusammensetzung behandelt, deren Viskosität durch Herabsetzung des Anteils an Verdickungsmitteln auf < 100 mPa.s bei 25 °C reduziert war.

Der Vergleich beider Haarhälften nach 20minütiger Einwirkung und anschließendem Ausspülen und Trocknen zeigte für die mit der erfindungsgemäßen Zusammensetzung behandelte Haarhälfte eine glänzende, kräftige rotbraune Färbung, während die mit der niedrigviskosen Zusammensetzung behandelte Haarhälfte lediglich einen matten, rötlich-braunen Farbton aufwies.

### Beispiel 5

Es wurde eine Rezeptur entsprechend der folgenden Zusammensetzung II hergestellt:

| | |
|---|---|
| Wasserstoffperoxid | 2,2 (Gew.-%) |
| Hydroxypropyltrimonium-Guar | 1,2 |
| Polyethylenglykol 10 000 | 6,0 |
| Polypropylenglykol-30/Polyethylenglykol-150 | 1,0 |
| Weizenproteinhydrolysat | 0,3 |
| Kaliumsorbat | 0,1 |
| Wasser | @ 100,0 |
| Viskosität bei 25 °C (Brookfield RVT, Spindel 4 bei 20 rpm): 3500 mPa.s pH-Wert: 6,8 | |

Diese Zusammensetzung wurde auf eine angefeuchtete Strähne aus mittelbraunem Menschenhaar aufgebracht, nach 20minütiger Einwirkung gespült und getrocknet.

Es wurde eine glänzende, hellbraune Farbe erhalten, das Haar besaß gute Kämmbarkeit, Volumen und Sprungkraft.

Eine analoge Zusammensetzung, die durch Weglassen des Verdickungsmittels lediglich eine Viskosität von 10 mPa.s bei 25 °C aufwies, ergab bei gleicher Behandlung eine unveränderte matte, hellbraune Farbe, deren Kämmbarkeit, Volumen und Elastizität gegenüber der mit dem erfindungsgemäßen Mittel behandelten Strähne stark abfiel.

### Beispiel 6

In einem weiteren Halbseitenversuch wurde das mittelblonde Haar einer Probandin in zwei Hälften geteilt, gewickelt und einer üblichen Dauerwellbehandlung unterzogen.

Anschließend wurde gespült.

Eine Hälfte wurde mit einer Fixierung der folgenden Zusammensetzung III behandelt:

| | |
|---|---|
| Wasserstoffperoxid | 2,6 (Gew.-%) |
| Dinatriumhydrogenphosphat | 0,2 |
| Polyquaternium-10 | 0,3 |
| Ethylenoxid/Propylenoxid-Kondensat | 0,5 |
| Polyethylenglykol 10-hydriertes Ricinusöl | 0,2 |
| Parfumöl | 0,2 |
| Distearyldimethylammoniumchlorid | 0,4 |
| Hydroxypropylmethylcellulose | 1,5 |
| Wasser | @ 100,0 |
| Phosphorsäure zur Einstellung auf pH 3,5 Viskosität bei 25 °C (Brookfield RVT, Spindel 4 bei 20 rpm): 2300 mPa.s | |

Die andere Hälfte wurde mit einer analogen Zusammensetzung behandelt, deren Viskosität allerdings nur 150 mPa.s bei 25 °C betrug. Nach 10minütiger Einwirkung wurde wiederum gespült, die Wickler entfernt und getrocknet.

Die mit der erfindungsgemäßen Zusammensetzung fixierte Haarhälfte zeigte einen hellen Glanz und war deutlich besser kämmbar sowie elastischer und lockerer als das mit der niedrigviskosen Zusammensetzung fixierte Haar.

## Patentansprüche

1. Verwendung eines Mittels, das 0,5 bis 12 Gew.-% Wasserstoffperoxid, berechnet auf die Gesamtzusammensetzung, in einer wäßrigen Grundlage enthält, sowie eine Viskosität von 1 000 bis 10 000 mPa.s bei 25°C (gemessen im Brookfield-Viskositmeter RVT mit Spindel Nr. 4 bei 20 rpm) und einen pH-Wert von 3 bis 7 aufweist, zur Aufhellung und Glanzverbesserung menschlicher Haare.

2. Verwendung nach Anspruch 1, dadurch gekennzeichnet, daß das Mittel eine Viskosität von 1 500 bis 5 000 mPa.s bei 25°C aufweist.

3. Verwendung eines Mittels nach Anspruch 1 oder 2 zum Färben von menschlichen Haaren, dadurch gekennzeichnet, daß das Mittel mindestens einen direktziehenden Haarfarbstoff enthält.

4. Verwendung eines Mittels nach Anspruch 3, dadurch gekennzeichnet, daß das Mittel 0,001 bis 2,5 Gew.-% mindestens eines direktziehenden Haarfarbstoffs, berechnet auf die Gesamtzusammensetzung des Mittels, enthält.

5. Verfahren zum Behandeln von dauergewelltem Haar, dadurch gekennzeichnet, daß mit einer Reduktionsmittel-Zusammensetzung verformtes Haar nach dem Spülen mit Wasser, gegebenenfalls nach Durchführung einer Zwischenbehandlung , in an sich üblicher Weise durch Aufbringen einer Oxidationsmittel-Zusammensetzung fixiert, anschließend eine Zusammensetzung, enthaltend 0,5 bis 12 Gew.-% Wasserstoffperoxid, berechnet auf die Gesamtzusammensetzung, mit einer Viskosität von 1 000 bis 10 000 mPa.s bei 25°C (gemessen im Brookfield-Viskosimeter RVT mit Spindel Nr. 4 bei 20 rpm), aufgebracht und nach fünf- bis dreißigminütiger Einwirkung desselben ausgespült wird.

## Claims

1. Use of a composition containing 0.5 to 12 % by weight hydrogen peroxide, based on the total composition, in an aqueous carrier, having a viscosity of 1,000 to 10,000 mPa.s at 25°C (measured in a Brookfield Viscosimeter RVT with spindle No. 4 at 20 rpm), for the brightening and gloss improvement of human hair.

2. Use according to claim 1, characterized in that the composition has a viscosity from 1,500 to 5,000 mPa.s at 25°C.

3. Use of a composition according to claims 1 or 2 for coloring of human hair, characterized in that the composition contains at least one direct-acting hair dye.

4. Use of a composition according to claim 3, characterized in that the composition contains 0.001 to 2.5 % by weight of at least one direct-acting hair dye, based on the total composition.

5. A process for the treatment of permanently waved hair, characterized in that hair deformed with a reducing agent composition is after rinsing with water, and, optionally an intermediate treatment, is fixed by the application of an oxidizing composition, and afterwards a composition containing 0.5 to 12 % by weight hydrogen peroxide, based on the total composition, with a viscosity of 1,000 to 10,000 mPa.s at 25°C (measured in a Brookfield Viscosimeter RVT with spindle No. 4 at 20 rpm), is applied onto the hair, and finally is rinsed out after 5 to 30 minutes' treatment.

## Revendications

1. Utilisation d'une composition qui, rapportée à la composition totale, contient 0,5 à 12 % en poids de peroxyde d'hydrogène dans une base aqueuse, et qui présente une viscosité comprise entre 1 000 et 10 000 mPa.s à 25 °C (mesurée au viscosimètre Brookfield RVT avec disque N° 4 à 20 tpm) et un pH de 3 à 7, pour l'éclaircissement et l'amélioration de la brillance de cheveux humains.

2. Utilisation selon la revendication 1, caractérisée en ce que la composition présente une viscosité comprise entre 1 500 et 5 000 mPa.s à 25 °C.

3. Utilisation d'une composition selon la revendication 1 ou 2, pour la teinture des cheveux humains, caractérisée en ce que la composition contient au moins un colorant capillaire direct.

4. Utilisation d'une composition selon la revendication 3, caractérisée en ce que la composition contient, rapporté à la composition totale de la composition, 0,001 à 2,5 % en poids d'au moins un colorant capillaire direct.

5. Procédé de traitement des cheveux permanentés, caractérisé en ce que des cheveux mis en forme avec une composition à base d'un agent réducteur, après rinçage à l'eau, et éventuellement après application d'un traitement intermédiaire, sont fixés de façon conventionnelle par application d'une composition à base d'un agent oxydant, sur lesquels est ensuite appliquée une composition contenant, rapporté à la composition totale, 0,5 à 12 % en poids de peroxyde d'hydrogène, avec une viscosité comprise entre 1 000 et 10 000 mPa.s à 25 °C (mesurée dans un viscosimètre Brookfield RVT avec disque N° 4 à 20 tpm), que l'on rince au terme d'un temps d'action de cinq à trente minutes.
